# FASCICULE DE BREVET EUROPEEN

(11) **EP 0 991 015 B1**
(45) Date de publication et mention de la délivrance du brevet: **01.12.2004**
(21) Numéro de dépôt: 99203096.5
(22) Date de dépôt: 21.09.1999
(51) Int. Cl.: G06T 5/00, A61B 19/00, G06F 19/00

(54) **Procédé de traitement d'images médicales d'ultrasons de structures osseuses et dispositif pour chirurgie assistée par ordinateur**
Bildverarbeitungsverfahren für medizinische Ultraschall-Abbildungen der Knochenstruktur, und ein Gerät für rechnerunterstützte Chirurgie
Method for processing ultrasonic medical images of bone structures, and an apparatus for computer assisted surgery

(30) Priorité: 29.09.1998 FR 9812160
(43) Date de publication de la demande: 05.04.2000
(73) Titulaire: Koninklijke Philips Electronics N.V., 5621 BA Eindhoven (NL)
(72) Inventeur: Lelong, Pierre, 75008 Paris (FR)
(74) Mandataire: Charpail, François

(56) Documents cités:
- EP-A- 0 629 963
- EP-A- 0 840 252
- WO-A-90/05494
- DE-A- 4 135 881
- FR-A- 2 550 403
- US-A- 4 722 056
- US-A- 5 457 754
- HAMADEH A ET AL: "Anatomy-based registration for computer-integrated surgery" COMPUTER VISION, VIRTUAL REALITY AND ROBOTICS IN MEDICINE, CVR MED'95, NICE, FRANCE, 3-6 APRIL 1995, pages 212-218, XP002107285 ISBN 3-540-59120-6, 1995, Berlin, Germany, Springer-Verlag

## Description

L'invention concerne un procédé de traitement d'image médicale d'ultrasons d'une structure non substantiellement transparente aux ondes ultrasonores, telle qu'une structure osseuse. L'invention concerne également un système de traitement d'image pour la mise en correspondance d'images obtenues par des systèmes différents d'acquisition d'images et un dispositif pour chirurgie assistée par ordinateur mettant en oeuvre ce procédé.

L'invention trouve son application dans l'industrie de fabrication d'appareils d'assistance chirurgicale par ordinateurs incluant un système d'acquisition d'images médicales, une station de travail et un système de visualisation d'images.

Un domaine d'application est celui des appareils d'acquisition d'images et systèmes de traitement d'images médicales pour l'aide à la chirurgie de la colonne vertébrale, ou bien de la chirurgie orthopédique, ou d'une manière générale de la chirurgie d'une structure osseuse. Dans ces cas, l'acte chirurgical peut comprendre l'insertion d'un outil linéaire tel qu'une vis, ou tel qu'une broche à l'intérieur de la structure osseuse. En prenant l'exemple de la chirurgie de la colonne vertébrale qui est des plus difficiles, un premier problème consiste à pratiquer l'opération sans endommager les nerfs, la moelle épinière et les vaisseaux. Des vis sont généralement insérées dans un pédicule vertébral sous un angle spécifique et enfoncées sous cet angle pour aller s'insérer dans le corps vertébral juste dans l'axe du pédicule. Cette opération requiert une localisation très précise de l'axe de pédicule. Dans la chirurgie non assistée par un système d'imagerie, on peut estimer que 30 % des vis pédiculaires sont incorrectement posées dans le cas des vertèbres lombaires, et que, à ce jour, l'opération des vertèbres dorsales et cervicales est impossible pour des raisons d'imprécision, parce que ces vertèbres sont beaucoup plus petites que les vertèbres lombaires. Un second problème est de réaliser aussi ce genre d'opération avec précision dans les cas où les structures osseuses sont sévèrement déformées et donc loin des connaissances A PRIORI déduites de statistique des formes des structures osseuses en cause, c'est-à-dire loin du modèle standard connu. Un troisième problème est que les structures osseuses ne se prêtent pas à la formation d'images avec certains types d'ondes. Il est possible d'en former des images de rayons X préopératoires qui sont confortables et très précises, tout en irradiant très peu le patient, mais il est difficile, voir impossible, d'opérer sous rayons X sous peine d'irradier trop fortement à la fois le patient et le chirurgien. Par ailleurs, on n'utilise généralement pas les ultrasons en relation avec les structures osseuses car on sait que les ondes ultrasonores ne pénètrent pas dans les structures osseuses, et que ces ondes montrent même des difficultés à traverser les ligaments qui lient les os entre eux aux endroits des articulations. Il est à ce jour difficile de visualiser par images d'ultrasons une articulation, ou des vertèbres, sans écarter du trajet du faisceau ultrasonore les ligaments en question, alors que cependant les ondes ultrasonores ne sont dangereuses ni pour le patient ni pour le chirurgien, et qu'à ce titre des images d'ultrasons peuvent être formées durant une opération.

Une méthode d'extraction de contours est déjà connue du brevet DE 41 35 881 (NOUR). Une image est acquise par un système de vidéo camera, ou par ultrasons, infrarouge, ou radiographie. L'image est numérisée. Les points de contour de l'image sont extraits par rapport au fond qui peut être noir ou blanc. Les points de contours sont ensuite reliés entre eux.

Il est déjà connu de la publication intitulée « Computer Assisted Spinal Surgery Using Anatomy-Based Registration » par Stéphane LAVALLEE, Philippe CINQUIN et alii, dans « COMPUTER-ASSISTED SPINE SURGERY », Article n° 32, pp.425-448, d'utiliser une prise de vue préopératoire obtenue par tomographie numérisée, qui est une technique d'imagerie de rayons X, en coopération avec un système référencé « Pointeur à Ultrasons » (FIG.32-12 de la publication citée). Le but est de mettre en correspondance une image d'ultrasons réalisée en cours d'intervention chirurgicale avec l'image de rayons X préopératoire, afin que le chirurgien puisse mettre en oeuvre, dans une zone repérée dans l'image d'ultrasons, un plan opératoire défini sur l'image de rayons X incluant la mise en place d'outils selon une localisation et une orientation appropriées. Pour obtenir la correspondance des images de rayons X et d'ultrasons, des dispositifs à diodes sont fixés d'une part à une structure osseuse du patient pour constituer une référence, et d'autre part à un transducteur d'un échographe à ultrasons, formant ainsi un système de repérage. Un algorithme de mise en correspondance (en anglais Matching Algorithm) est ensuite utilisé pour faire coïncider la structure osseuse représentée dans l'image de rayons X préopératoire avec des points situés sur l'onde de front réfléchie par la même structure osseuse représentée dans l'image d'ultrasons acquise durant l'opération.

Ce procédé connu de la publication citée permet d'acquérir des petits morceaux de courbes planaires sur l'image d'ultrasons, basé sur le fait que la partie supérieure des bords épais représentés dans cette image d'ultrasons est le vrai bord de la structure osseuse parce qu'elle correspond au front de l'onde réfléchie par ce bord osseux, en premier issu du transducteur qui est positionné à la partie supérieure de l'image. Ce procédé connu a été seulement expérimenté sur des échantillons de vertèbres, hors du corps d'un patient. Dans l'hypothèse d'une application à une opération réelle d'un patient, une difficulté réside dans le fait que ce procédé connu n'est pas automatique. Il est en effet difficile de guider une opération aussi délicate en utilisant un procédé qui n'est pas automatique et qui n'est pas mené à bien en temps réel. La publication citée ne donne pas d'indications pouvant conduire à des moyens d'automatisation.

Le cadre de l'invention est défini par les revendications

La présente invention a pour but de fournir un procédé de traitement d'une image d'ultrasons d'une structure non substantiellement transparente aux ultrasons, telle qu'une structure osseuse, ayant des étapes d'extraction automatique de points du contour de ladite structure, ces étapes étant en outre mise en oeuvre en temps réel.

Selon l'invention ces buts sont atteints au moyen d'un procédé de traitement d'images ultrasonores selon la revendication 1.

Le procédé proposé selon invention présente plusieurs avantages. Il fournit, dans une image d'ultrasons un contour plus précis que le procédé connu, et un contour de longueur substantiellement plus grande par rapport au champ de l'image d'ultrasons, ce qui permet de réaliser une meilleure correspondance de l'image préopératoire et de l'image d'ultrasons. En outre, comme ce procédé peut être mis en oeuvre automatiquement et en temps réel d'après les données d'images prélevées sur le site réel, la position de ce contour précis peut être mise à jour par un système selon l'invention durant l'opération chirurgicale, d'où il résulte que la mise en correspondance de image d'ultrasons et de l'image préopératoire peut être rafraîchie de façon appropriée.

Dans une application, on propose un système de mise en correspondance d'images acquises à des instants différents avec des systèmes d'acquisition différents pour s'appliquer dans un système d'imagerie médicale pour chirurgie assistée par ordinateur, en particulier dans un système d'imagerie médicale pour une opération chirurgicale dans le domaine de la chirurgie osseuse

Un problème lié à un tel système de mise en correspondance d'images réside dans le fait que l'étape d'acquisition d'images d'ultrasons fournit des informations relatives à l'onde de front réfléchie par la structure osseuse qui sont peu précises, voir inexistantes, parce que beaucoup d'autres structures diverses non osseuses, mais aussi non transparentes aux ultrasons, recouvrent la structure osseuse et perturbent ou bien empêchent la réflexion des ultrasons. Il en résulte que, dans ce cas, l'étape de mise en correspondance de l'image d'ultrasons et de l'image préopératoire peut être peu précise. Selon l'invention, le système de mise en correspondance d'images pour l'aide à la chirurgie proposée selon la revendication 6 permet de résoudre ce problème.

Dans une autre application, on propose un dispositif pour chirurgie assistée par ordinateur qui comprend des moyens pour mettre en oeuvre une telle méthode de mise en correspondance d'images dans le domaine de la chirurgie osseuse.

L'invention est décrite ci-après en détail, en référence aux FIGs schématiques annexées qui comprennent :
la FIG.1A représentant le déroulement des étapes du procédé de traitement d'images d'ultrasons US sous forme d'un diagramme de blocs fonctionnels, et la FIG.1B représentant le déroulement de la méthode de chirurgie assistée par ordinateur qui peut être effectuée en utilisant les systèmes selon les revendications 6-8;
la FIG.2A illustrant l'étape d'extraction de maxima locaux d'intensité sur une colonne dans l'image numérisée d'ultrasons US et la FIG.2B montrant une courbe d'intensité G correspondante sur une telle colonne,
la FIG 3 illustrant la sélection de chaînes dans l'image numérisée d'ultrasons US,
la FIG.4A montrant une surface de structure osseuse (vertèbre) extraite d'une image CT, la FIG.4B représentant une image d'ultrasons US, la FIG.4C montrant une image d'ultrasons US après extraction de points du contour de la structure osseuse et la FIG.4D montrant le résultat de la mise en correspondance des images US et CT traitées,
La FIG.5 illustrant un dispositif pour chirurgie assistée par ordinateur.

L'invention concerne un procédé de traitement d'images d'ultrasons. L'invention concerne aussi un système mettant en oeuvre une méthode de mise en correspondance d'images pour l'assistance opératoire incluant des étapes de ce procédé. L'invention concerne en outre un dispositif d'assistance opératoire par ordinateur, dans le domaine de la chirurgie osseuse, ayant des moyens pour mettre en oeuvre cette méthode.

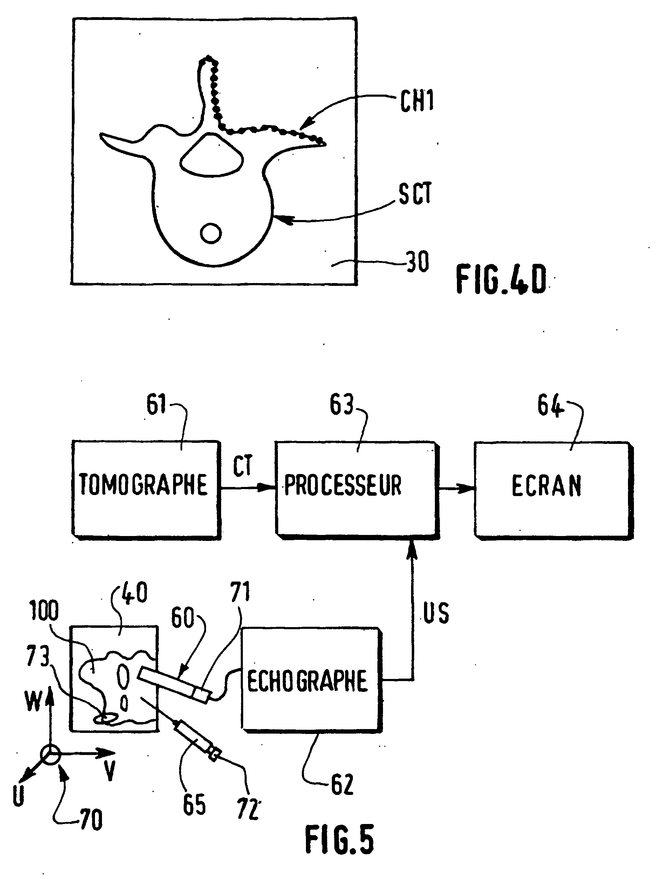

Comme illustré par des blocs fonctionnels sur la FIG.5, le dispositif pour chirurgie assistée par ordinateur comporte des moyens pour fournir une localisation et une orientation précises d'outils chirurgicaux 65 utilisés durant une opération chirurgicale, par rapport à une structure osseuse 100 située dans un champ opératoire 40 et pour fournir une visualisation de ces outils chirurgicaux avec leur localisation et orientation précises dans une image haute définition tridimensionnelle correspondant au champ opératoire. En particulier, ce dispositif comprend des moyens d'acquisition de données d'images médicales reliés à un processeur mettant en oeuvre les étapes d'une méthode de mise en correspondance d'images pour l'assistance opératoire et un système d'affichage d'images médicales relié au processeur. Les moyens d'acquisition de données d'images comprennent au moins des moyens 61 d'acquisition d'une image préopératoire haute définition notée CT d'une structure osseuse à opérer et des moyens 60, 62 d'acquisition d'une image d'ultrasons US intra-opératoire d'une zone de la même structure osseuse 100 à opérer. L'acquisition de l'image tridimensionnelle préopératoire haute définition CT de la structure osseuse à opérer peut être fournie notamment par un appareil 61 de résonance magnétique ou par un appareil 61 de tomographie numérisée basée sur des images de rayons X. L'acquisition de l'image d'ultrasons US peut être réalisée au moyen d'un transducteur ultrasonore qui peut être une sonde standard 60 couplée à un échographe 62. Ce dispositif d'assistance opératoire comprend aussi des moyens d'affichage 64, par exemple un écran, pour effectuer la visualisation des outils dans l'image haute définition. Ce dispositif peut aussi comprendre des moyens d'enregistrement. Cette visualisation peut être obtenue par une représentation simultanée durant l'opération chirurgicale, sur l'écran 64, de l'image tridimensionnelle préopératoire CT et des outils 65 sous forme d'objets virtuels en trois dimensions. Pour réaliser cette représentation simultanée, ce dispositif comprend d'une part des moyens 70, 71, 72, 73 pour déterminer avec précision la localisation et les orientations en trois dimensions des outils chirurgicaux réels 65 utilisés et manipulés par le chirurgien durant l'opération vis-à-vis de la structure osseuse à opérer et d'autre part un processeur 63 pour transmettre aux outils virtuels la localisation et les orientations en trois dimensions des outils réels et permettre au chirurgien de visualiser sur l'image haute définition la localisation et les orientations de ces outils réels appliqués selon un plan opératoire approprié. Les moyens de localisation et de détermination des orientations de la structure osseuse et des outils réels durant l'opération consistent en un système de repérage qui comprend un dispositif de repérage fixe 70 dans la salle d'opération, un dispositif de repérage 73 associé à la structure osseuse, un dispositif de repérage 71 associé au transducteur ultrasonore 60 et un dispositif de repérage 72 associé à chaque outil 65 utilisé. La localisation et les orientations des dispositifs de repérage associés à la structure osseuse, au transducteur et aux outils réels sont déterminées par rapport au dispositif de repérage fixe 70. Les dispositifs de repérage sont favorablement du type à trois diodes LEDs. De tels dispositifs à diodes sont connus de l'homme du métier. Parmi les outils chirurgicaux 65, il peut y avoir une perceuse, des broches, des vis, etc. Le microprocesseur 63 est un élément important du dispositif d'assistance opératoire qui gère et qui met en oeuvre une méthode de traitement d'image appliquée à l'assistance opératoire pour mettre l'image préopératoire CT en coïncidence avec le champ opératoire 40.

Un dispositif mettant en oeuvre cette méthode de traitement d'image appliqué à l'assistance opératoire comprend selon l'invention des moyens exécutant des étapes de traitement automatique et en temps réel de l'image d'ultrasons et est décrit ci-après à titre d'exemple dans une application au traitement images de la colonne vertébrale. Le concept de chirurgie guidée par ordinateur a pour avantage de rendre la chirurgie de la colonne vertébrale plus précise et plus sûre. Le dispositif d'assistance opératoire a pour but d'acquérir l'image d'ultrasons US dans le système de repérage et de mettre en correspondance l'image préopératoire CT et l'image d'ultrasons US pour faire coïncider l'image préopératoire CT avec le champ opératoire 40.

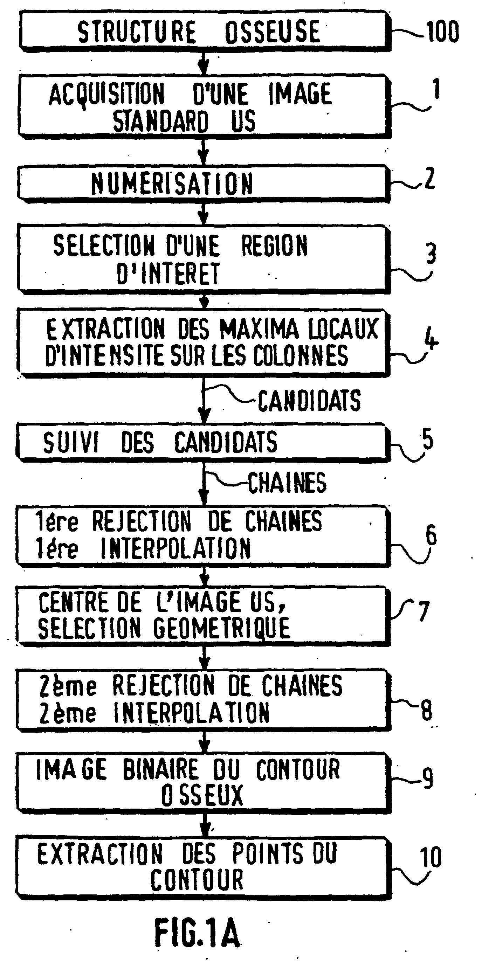

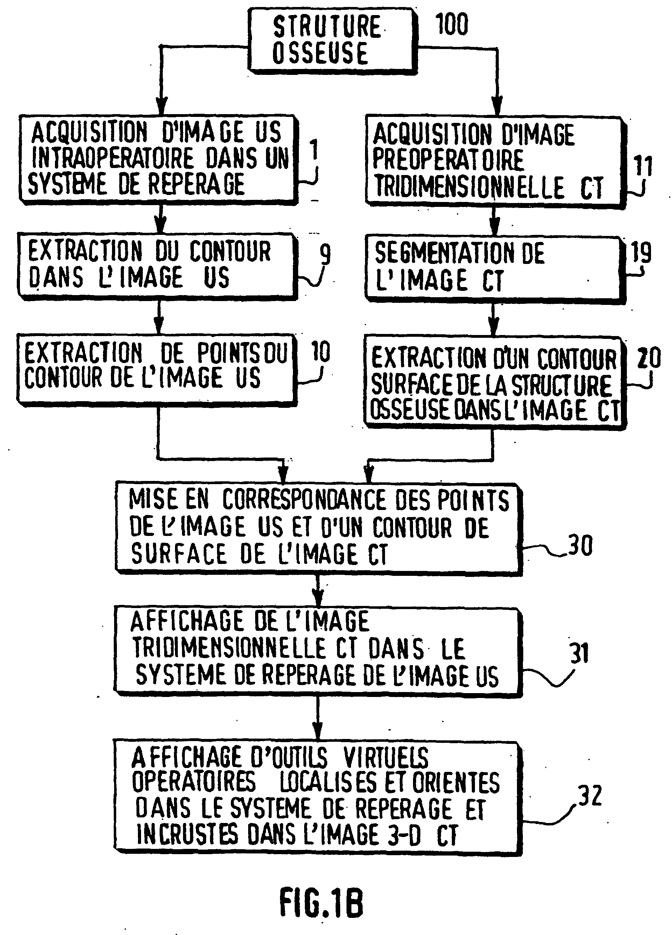

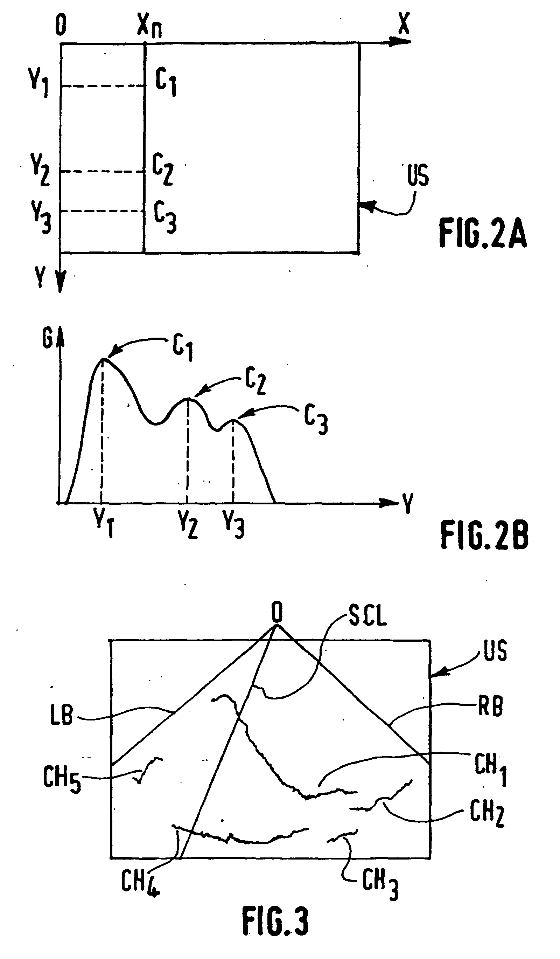

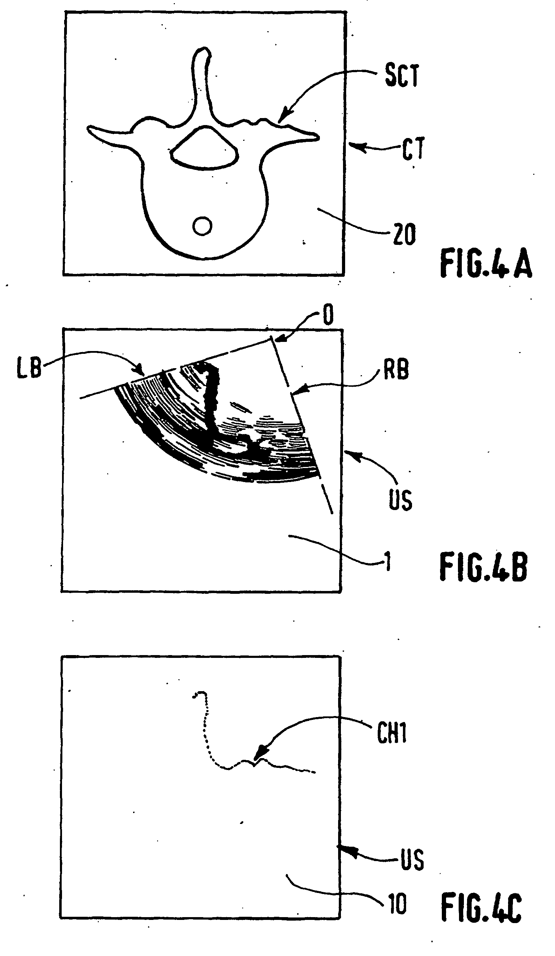

Comme représentée par les blocs fonctionnels de la FIG. 1B, une méthode d'assistance opératoire qui peut être effectuée en utilisant un dispositif selon l'invention comprend des étapes de :
a) Acquisition 11 d'une image numérisée préopératoire tridimensionnelle haute définition, notée CT, de la structure osseuse à opérer, en utilisant les moyens d'acquisition d'images 61.
b) Segmentation 19 de cette image CT par des moyens connus d'extraction de contours.
c) Extraction 20 de la surface de la structure osseuse dans image CT segmentée. La structure osseuse est alors représentée par un volume binaire de voxels ayant des valeurs d'intensité 0 ou 1 selon qu'ils appartiennent ou non à la surface de cette structure. La surface extraite notée SCT est illustrée par la FIG.4A.
d) Mise en oeuvre d'un procédé de traitement d'image ultrasons d'une zone de la même structure osseuse induant spécifiquement l'extraction de points du contour de la structure osseuse localisés dans le système de référence, ce procédé étant illustré par les blocs fonctionnels de la FIG.1A et incluant des étapes de :
   1) Acquisition 1 d'une image d'ultrasons notée US d'une zone de la structure osseuse, c'est-à-dire d'une partie de vertèbre, au commencement de l'opération chirurgicale. A cet effet le chirurgien expose la structure osseuse à opérer et réalise, au moyen du transducteur 60 associé à l'échographe 62, une acquisition d'au moins une image d'ultrasons standard. Cette acquisition d'image est faite par exemple en emplissant la cavité formée par le champ opératoire avec un produit de couplage, par exemple du sérum physiologique, et en positionnant le transducteur 60 avec son extrémité 67 immergée dans le sérum de manière à obtenir un bon couplage avec la structure osseuse 100 soumise aux ondes ultrasonores. Après l'acquisition de l'image ou d'une séquence d'images d'ultrasons, le produit de couplage peut être éliminé par aspiration pour que le chirurgien poursuive l'opération chirurgicale en utilisant des outils réels 65. Les images d'ultrasons obtenues sont à ce jour bidimensionnelles. Pour obtenir une image tridimensionnelle, il faut enregistrer la position géométrique du transducteur 60 et construire une image tridimensionnelle en combinant plusieurs images bidimensionnelles US en tenant compte des informations relatives à leurs positions respectives. A cet effet, durant l'acquisition d'une image US, le système de repérage 70, 71, 73 enregistre la position de la sonde 60 dans un système de coordonnées orthogonales, son orientation dans l'espace par des angles vis-à-vis des axes de coordonnées en même temps que l'image correspondante d'ultrasons est enregistrée par le dispositif 62. Le transducteur 60 est par exemple incliné par rapport à la vertèbre d'un angle d'environ 25 degrés, et son extrémité 67 immergée dans le produit de couplage est positionnée à environ 1cm de la structure osseuse 100. Le transducteur effectue un balayage mécanique de la zone opératoire 40 qui produit plusieurs images d'ultrasons US, par exemple une centaine. La vertèbre peut être balayée mécaniquement sur deux côtés. Les images d'ultrasons sont acquises sous forme analogique, puis sont numérisées par des moyens appropriés 2. Une image d'ultrasons standard analogique est usuellement obtenue par balayage ultrasonore selon des lignes qui forment des rayons notés SCL à partir d'un point O où est localisé le transducteur ultrasonore qui émet les ondes ultrasonores et reçoit les échos renvoyés par des structures situées sur le trajet de ces rayons. Ces images standards sont constituées de points ayant un niveau d'intensité relatif à l'écho renvoyé par la structure localisée au point correspondant sur le rayon SCL.
   2) Numérisation 2 de l'image standard. Une image d'ultrasons numérisée est rectangulaire et est formée des points d'une image standard maintenant repérés dans un repère orthogonal avec des coordonnées X pour les colonnes et Y pour les lignes, et avec leur intensité d'origine et leur localisation d'origine dans l'image standard. L'image d'ultrasons numérisée ainsi acquise est illustrée par la FIG.4B.
      Un but du dispositif mettant en oeuvre la méthode de mise en correspondance d'images pour l'assistance opératoire est de faire coïncider des points de contour de la structure osseuse de l'image d'ultrasons, dont on sait déterminer la position et l'orientation par rapport au champ opératoire, avec un contour correspondant de la structure osseuse dans l'image préopératoire CT, pour déterminer ainsi la position et l'orientation de cette image CT. Un autre but est d'afficher l'image CT, qui présente l'avantage d'être beaucoup plus précise et confortable que l'image d'ultrasons US, sur l'écran 64, durant le déroulement de l'opération chirurgicale. Un autre but est d'incruster des instruments virtuels reproduisant, dans cette image CT, la forme et les mouvements des instruments réels 65, en temps réel. Il est donc de la plus haute importance d'extraire de l'image US un nombre de points appropriés pour déterminer de la façon la plus précise possible la surface de la vertèbre qui fait face au transducteur.
      Un dispositif mettant en oeuvre le procédé de traitement d'images d'ultrasons spécifique qui est décrit ici est apte à atteindre les buts cités plus haut. Ce procédé présente l'avantage de fournir des points définissant des contours substantiellement longs et continus de la vertèbre d'une manière complètement automatique et en temps réel. Comme représenté par les blocs fonctionnels de la FIG.1A, ce procédé se continue par le déroulement des étapes suivantes qui sont mises en oeuvre par des moyens de calculs et de gestion des étapes, tel que le processeur 63 :
   3) Délimitation 3 d'une zone d'intérêt dans une région de vertèbre présentant des inégalités de surface, c'est-à-dire des détails caractéristiques, afin de fournir le plus possible d'informations utiles pour le positionnement respectifs des images CT et US. En outre la zone d'intérêt est choisie de surface limitée pour inclure le moins possible de tissus mous et pour ne pas inclure de structures mobiles les unes vis-à-vis des autres.
   4) Extraction 4 de points appelés « meilleurs candidats » dans l'image d'ultrasons numérisée effectuée selon les sous-étapes suivantes :
      4-1) Sélection de points appelés "candidats". A cet effet, un balayage de l'image US numérisée est effectué colonne par colonne comme montré sur la FIG.2A qui représente le balayage d'une colonne Xn sur laquelle on trouve des maxima d'intensité C1, C2, C3 aux positions Y1, Y2, Y3. Ces points « candidats » sont sélectionnés lorsqu'ils présentent localement un maximum d'intensité le long de cette colonne, comme illustré par la FIG.2B qui représente les valeurs des maxima d'intensité G trouvées le long de la colonne Xn aux positions Y1, Y2, Y3. Pour trouver des maxima consistants, il est préférable d'opérer, antérieurement à cette opération de sélection, un filtrage passe-bas ayant pour effet de diminuer le bruit dans l'image US numérisée.
      4-2) Seuillage d'intensité. On effectue un seuillage pour éliminer un certain nombre de poins candidats et pour conserver des points ayant un niveau d'intensité au-dessus de ce seuil et appelés points «meilleurs candidats ».
   5) Suivi 5 des points meilleurs candidats (en anglais Tracking) dans l'image US numérisée pour les lier entre eux et former des chaînes effectué dans les conditions suivantes :
      Condition 1 : On définit une direction de suivi, par exemple de gauche à droite et on examine les points meilleurs candidats.
      Condition 2 : Tout point qui n'a pas encore été admis dans une chaîne, démarre une nouvelle chaîne à condition de ne pas avoir été par ailleurs rejeté.
      Condition 3 : On recherche et on sélectionne le point meilleur candidat appelé meilleur voisin sur les colonnes suivantes situées de proche en proche, à droite (du fait de la condition 1), et à une distance inférieure à une distance prédéterminée.
      Condition 4 : On recherche et on sélectionne les meilleurs voisins de la gauche vers la droite dans des voisinages de rayons croissants mais ayant un maximum prédéterminé et sur des colonnes.
      Condition 5 : Tous les points meilleurs candidats qui ne sont pas indus dans une chaîne sont éliminés.
   6) Première sélection 6 des chaînes illustrée par la FIG.3 qui montre une image US dans laquelle on a déterminé plusieurs chaînes CH1, CH2, CH3, CH4, CH5 dans les conditions :
      Condition 6 : Toute chaîne inférieure à une longueur de référence mesurée en points est éliminée. Par exemple la longueur de référence est : 8 points.
      Condition 7 : Lorsque les chaînes comprennent des lacunes de peu de points (par exemple 1 point), ces lacunes sont comblées par interpolation. Par exemple dans le cas où une colonne est vide de point, on lui attribue un point localisé en réalisant une interpolation linéaire entre les points répondant à la condition 3, situés sur la colonne de gauche et sur la colonne de droite de la colonne vide.

      On peut parler indifféremment de conditions ou de critères.
   7) Sélection géométrique 7 pour déterminer le contour des structures osseuses et éliminer les zones internes illustrée par la FIG.3.
      7-1) Détermination du point centre O de image échographique US numérisée. L'image échographique US est caractérisée par les lignes radiales notées SCL de balayage des ultrasons à partir du centre O qui correspond au transducteur ultrasonore. Ce centre O est déterminé avec précision, à partir de cette image d'ultrasons US elle-même, à l'intersection des lignes radiales des bords gauche LB et droit RB.
      7-2) Sélection géométrique proprement dite comprenant un examen de la localisation des chaînes telles que CH1 à CH5 trouvées dans l'étape précédente de suivi dans l'image US numérisée. Chaque ligne radiale de balayage SCL est considérée à partir du point centre O, en s'éloignant du centre O, par une opération de balayage radial orienté de l'image échographique dans laquelle, chaque premier point détecté appartenant à la fois à une ligne de balayage radial et à une chaîne est gardé. Cette étape permet de déterminer le front de l'onde échographique et permet donc de sélectionner les points de contour de la structure osseuse vis-à-vis des points de l'intérieur de la structure osseuse ou d'autres structures. Par exemple, sur la FIG.3, on va garder les chaînes CH1 et CH2.
   8) Seconde sélection 8 des chaînes, selon le principe déjà décrit qui élimine les chaînes trop courtes, par exemple de longueur inférieure à 8 points. Cette sélection est suivie d'une seconde interpolation : s'il se trouve encore des lacunes sur les chaînes, on les comble par exemple selon le principe déjà décrit d'interpolation linéaire.
   9) Affichage 9 de l'image des contours des structures osseuses qui est une image binaire comprenant les chaînes correspondant au contour extérieur par exemple des vertèbres, c'est-à-dire le contour qui est en vis-à-vis du transducteur ultrasonore. Par exemple sur la FIG.4C, après les étapes de sélection, seule est gardée la chaîne CH1.
   10) Extraction 10 de points de contours. Ces points seront utilisés pour réaliser la mise en correspondance avec l'image CT.

   La méthode de mise en correspondance d'images pour l'assistance opératoire se poursuit par des étapes de :
e) Calibrage et mise à la même échelle des images CT et US. Cette étape est effectuée par tout moyen approprié connu. On suppose en première approximation que ces images sont dépourvues de distorsions.
f) Mise en correspondance 30 de l'image préopératoire CT et de l'image US des contours. La mise en correspondance de l'image des contours fournie par le traitement de l'image préopératoire CT avec l'image des points de contours fournie par le traitement de l'image US numérisée est effectuée au moyen de tout algorithme approprié de mise en correspondance d'images connu de l'état de la technique qui converge en fournissant une image des contours de l'image CT et des points de contours de l'image US superposés. Les contours extraits et déterminés dans l'image US selon le procédé de l'invention sont substantiellement longs et très précis. Dans ces conditions, la convergence de l'algorithme est rapide et fournit une localisation précise des structures osseuses de l'image préopératoire CT en trois dimensions par rapport au champ opératoire dans le système de repérage fixe. Cette étape est illustrée par la FIG.4D qui montre la mise en coïncidence de la chaîne CH1 de l'image US avec la surface extraite SCT de l'image CT.
g) Représentation 31 sur un écran 64 de l'image préopératoire haute définition et tridimensionnelle CT en coïncidence avec le champ opératoire ; et affichage 32 d'outils chirurgicaux virtuels représentant des outils réels 65. Les outils virtuels sont incrustés dans cette image 31. Les outils chirurgicaux réels 65 sont munis de dispositifs de repérage 72 à diodes LEDs qui donnent des mesures de leurs positions et de leur orientations dans le système de coordonnées orthogonales défini précédemment. D'après ces mesures, les outils virtuels sont positionnés et orientés en trois dimensions vis-à-vis de la structure osseuse de l'image préopératoire tridimensionnelle haute définition

En utilisant un dispositif mettant en oeuvre cette méthode de mise en correspondance d'images pour l'assistance chirurgicale, le chirurgien peut positionner les outils chirurgicaux réels avec la précision requise vis-à-vis de la structure osseuse réelle grâce à la visualisation précise en trois dimensions de ces outils virtuels. Donc, certaines opérations chirurgicales qui n'étaient jusqu'à ce jour pas envisageables, deviennent possibles.

## Revendications

1. Procédé de traitement d'images incluant une étape d'acquisition, au moyen d'un transducteur ultrasonore couplé à un dispositif échographique, d'une image échographique numérisée d'une structure non substantiellement transparente aux ondes ultrasonores et une étape d'extraction de points de contour de ladite structure, selon lequel l'étape d'extraction des points est automatisée au moyen des sous-étapes de :
détection (4), sur des colonnes de points de l'image échographique numérisée, des points d'intensité localement maximale le long de chaque colonne,
chaînage (5) des points détectés d'une colonne à une autre dans un voisinage prédéterminé,
Sélection (6-10) des chaînes dans l'image échographique standard en déterminant la chaîne la plus proche du transducteur ultrasonore comme contour de ladite structure.

2. Procédé selon la revendication 1, selon lequel l'étape de chaînage est effectuée selon des critères de :
définition d'un seuil d'intensité pour les points chaînés,
définition d'une direction de chaînage en partant d'une première colonne vers une dernière colonne d'un bord à un bord opposé de l'image numérisée,
définition du voisinage comme une distance de référence dans une fourchette prédéterminée, pour chaîner deux points sur des colonnes différentes.

3. Procédé selon la revendication 2, selon lequel l'étape de chaînage est effectuée selon les critères supplémentaires de :
création d'un départ d'une chaîne nouvelle avec tout point qui ne répond pas au critère de voisinage précédent,
définition d'une longueur minimale des chaînes pour conserver les points chaînés,
élimination des points n'appartenant à aucune chaîne.

4. Procédé selon la revendication 3, selon lequel l'étape de chaînage comprend en outre une opération d'interpolation pour combler sur les chaînes des lacunes inférieures à une distance prédéterminée.

5. Procédé selon l'une des revendications 1 à 4 selon lequel la détermination de la localisation du transducteur est effectuée dans l'image échographique numérisée, qui montre des lignes radiales de balayage ultrasonore, par une détermination de l'intersection des deux lignes radiales extrêmes de cette image.

6. Un système de traitement d'image incluant un processeur (63) ayant accès à des données d'images et muni de moyens pour effectuer une mise en correspondance d'images acquises à des instants différents avec des moyens différents d'acquisition d'images, ces moyens comprenant des instructions pour effectuer:
a) une acquisition à un premier instant, avec des premiers moyens d'acquisition d'images, d'une première image, de résolution substantiellement élevée d'une zone d'une de structure d'intérêt non substantiellement transparente aux ondes ultrasonores,
b) une segmentation de ladite première image pour déterminer un contour de la structure d'intérêt,
c) une acquisition à un second instant, au moyen d'un système d'acquisition d'image échographique, d'au moins une image échographique numérisée d'une zone de la même structure d'intérêt non substantiellement transparente aux ondes ultrasonores, cette image échographique étant associée à des mesures de localisation et orientation dans un système de repérage ayant un repère fixepar rapport au système de formation d'image échographique,
d) une extraction automatique de points de contour, selon l'une des revendications 1 à 5, de l'image échographique numérisée pour fournir des points de contour de la structure d'intérêt avec leurs localisations et orientations dans le système de repérage ;
e) une mise en correspondance du contour de la structure d'intérêt de ladite première image segmentée et des points de contour extraits de l'image échographique pour localiser et orienter ladite première image dans le système de repérage,
f) une représentation simultanée de ladite première image -repérée dans le système de repérage et des outils chirurgicaux sous forme d'images virtuelles incrustées dans cette image représentant des outils chirurgicaux réels utilisés durant une opération avec leurs localisations et orientations actuelles déterminées dans le système de repérage.

7. Dispositif pour chirurgie assistée par ordinateur pour une opération chirurgicale d'une structure d'intérêt comprenant :
un système d'acquisition de données d'images, incluant un dispositif (61) d'acquisition d'image haute résolution et un dispositif (62) d'acquisition d'image échographique,
un système de repérage comprenant un dispositif de repérage fixe, un dispositif de repérage de la structure osseuse d'intérêt non substantiellement transparente aux ondes ultrasonores, un dispositif de repérage de moyen d'acquisition de l'image échographique, et un dispositif de repérage d'outils mobiles dans le champs de l'image échographiques par rapport au repère fixe,
un système de traitement d'image selon la revendication 6 incluant un processeur ayant accès aux données d'images et muni de moyens comprenant des instructions pour effectuer une mise en correspondance d'images,
et un système d'affichage et occasionnellement d'enregistrement des données d'image acquises et traitées par ladite méthode, relié au système de traitement d'image.

8. Dispositif pour chirurgie assistée par ordinateur, pour une opération chirurgicale où la structure non substantiellement transparente aux ondes ultrasonores est-une structure osseuse, selon la revendication 7, dans lequel les dispositifs d'acquisition de données d'image comprennent :
des moyens d'acquisition d'une image préopératoire de résolution substantiellement élevée d'une structure osseuse d'intérêt sélectionnée pour une opération chirurgicale,
des moyens de segmentation de image préopératoire pour déterminer un contour de la structure osseuse d'intérêt,
et des moyens d'acquisition, durant une opération, d'au moins une image échographique numérisée d'une zone de la même structure osseuse, cette image étant associée à des mesures de localisation et orientation dans le système de repérage ayant un repère fixe,
et dans lequel le système de traitement images comprend un microprocesseur ayant accès aux données d'images et qui est munis de moyens pour effectuer :
une extraction automatique de points de contour, selon l'une des revendications 1 à 5, de limage échographique numérisée pour fournir des points de contour de la structure osseuse d'intérêt en temps réel durant l'opération avec leurs localisations et orientations dans le système de repérage ;
une mise en correspondance du contour de la structure osseuse d'intérêt de l'image préopératoire segmentée et des points de contour extraits de l'image échographique pour localiser et orienter l'image préopératoire dans le système de repérage, et dans lequel le microprocesseur est aussi muni de moyens pour transmettre au système d'affichage :
une représentation simultanée de l'image préopératoire repérée dans le système de repérage et des outils chirurgicaux sous forme d'images virtuelles incrustées dans cette image représentant des outils chirurgicaux réels utilisés durant l'opération avec leurs localisations et orientations actuelles déterminées dans le système de repérage.

## Patentansprüche

1. Verfahren zur Bildverarbeitung mit einem Schritt zur Aufnahme mittels eines an ein Echographiegerät gekoppelten Ultraschall-Transducers eines digitalisierten Echographiebilds einer für Ultraschall nicht grundsätzlich durchlässigen Struktur und einem Schritt zur Extraktion der Konturpunkte der besagten Struktur, bei dem der Schritt zur Extraktion der Punkte mittels folgender Unterschritte automatisiert ist:
Detektion (4) in den Spalten der digitalisierten Echographiebildpunkte derjenigen Punkte mit lokaler maximaler Intensität entlang jeder Spalte,
Verkettung (5) der von einer Spalte zu anderen erkannten Punkte in einer vorbestimmten Nachbarschaft,
Auswahl (6-10) der Ketten im Standard-Echographiebild mit der Bestimmung der am nächsten am Ultraschall-Transducer gelegenen Kette als Kontur der besagten Struktur.

2. Verfahren nach Anspruch 1, nach dem der Schritt zur Verkettung nach folgenden Kriterien ausgeführt wird:
Definition eines Intensitätsgrenzwerts für die verketteten Punkte,
Definition einer Verkettungsrichtung ausgehend von einer ersten Spalte zu einer letzten Spalte von einem Rand zu einem gegenüberliegenden Rand des digitalisierten Bildes,
Definition der Nachbarschaft als Bezugsabstand in einer vorbestimmten Spanne zur Verkettung zweier Punkte über verschiedene Spalten.

3. Verfahren nach Anspruch 2, nach dem der Schritt zur Verkettung nach folgenden zusätzlichen Kriterien ausgeführt wird:
Schaffung einerseits einer neuen Kette mit jedem Punkt, der nicht den vorhergehenden Nachbarschaftskriterien entspricht,
Definition einer minimalen Kettenlänge für den Erhalt der verketteten Punkte,
Aussonderung derjenigen Punkte, die zu keiner Kette gehören.

4. Verfahren nach Anspruch 3, nach dem der Schritt zur Verkettung außerdem eine Interpretationsoperation beinhaltet, um in den Ketten diejenigen Lücken zu füllen, die unter einem vorbestimmten Abstand liegen.

5. Verfahren nach einem der Ansprüche 1 dies 4, nach dem die Bestimmung der Lokalisierung des Transducers im digitalisierten Echographiebild ausgeführt wird, das radiale Ultraschall-Abtastzeilen mit der Bestimmungen der Schnittstelle der zwei äußersten radialen Linien dieses Bilds zeigt.

6. System zur Bildverarbeitung mit einem Prozessor (63) mit Zugriff auf Bilddaten und mit Mitteln zur Ausführung einer Gleichsetzung von Bildern versehen, die in verschiedenen Zeitpunkten mit verschiedenen Bildaufnahmemitteln aufgenommen wurden, wobei diese Mittel Anweisungen enthalten, um folgendes auszuführen:
a) Aufnahme in einem ersten Zeitpunkt mit den ersten Bildaufnahmemitteln eines ersten Bildes grundsätzlich hoher Auflösung einer Zone einer Interessenstruktur, die grundsätzlich für Ultraschallwellen nicht durchlässig ist;
b) Segmentierung des besagten ersten Bilds zur Bestimmung einer Kontur der Interessenstruktur;
c) Aufnahme in einem zweiten Zeitpunkt mit einen Echographiebild-Aufnahmesystem von mindestens einem digitalisierten Echographiebild einer Zone selber Interessenstruktur, die grundsätzlich für Ultraschallwellen nicht durchlässig ist, wobei dieses Echographiebild mit den Messungen zur Lokalisierung und Orientierung in ein Bezugssystem mit einem festen Bezug zum Echographiebild-Aufnahmesystem eingebunden wird;
d) automatische Extraktion der Konturpunkte des digitalisierten Echographiebilds nach einem der Ansprüche 1 bis 5 für die Ausgabe der Konturpunkte der Interessenstruktur mit ihren Lokalisierungen und Orientierungen im Bezugssystem;
e) Gleichsetzung der Kontur der Interessenstruktur des besagten ersten segmentierten Bildes und der extrahierten Konturpunkte des Echographiebilds zur Lokalisierung und Orientierung des besagten ersten Bilds im Bezugssystem;
f) gleichzeitige Darstellung des besagten ersten im Bezugssystem georteten Bilds und der chirurgischen Werkzeuge in der Form von verankerten virtuellen Bildern in diesem Bild, das während einer Operation verwendete reelle chirurgische Werkzeuge mit ihren aktuellen im Bezugssystem festgelegten Lokalisierungen und Orientierungen darstellt.

7. Gerät für rechnerunterstützte Chirurgie für eine chirurgische Operation einer Interessenstruktur mit:
einem System zur Aufnahme von Bilddaten mit einem Gerät (61) zur Aufnahme von hoch aufgelösten Bildern und einem Gerät (62) zur Aufnahme von Echographiebildern,
einem Ortungssystem mit einem festen Ortungsgerät, einem Gerät zur Ortung der Knochenstruktur von Interesse, die grundsätzlich für Ultraschallwellen nicht durchlässig ist, einem Gerät zur Ortung der Aufnahmemittel des Echographiebilds und ein Gerät zur Ortung von mobilen Instrumenten in dem Echographiebild hinsichtlich des festen Bezugs,
einem System zur Bildverarbeitung nach Anspruch 6 mit einem Prozessor mit Zugriff auf Bilddaten und mit Instruktionsmitteln versehen, um eine Gleichsetzung von Bildern auszuführen, und
einem System zur Anzeige und gelegentlich zur Aufzeichnung von Bilddaten, die mit der besagten mit dem System zur Bildverarbeitung verbundenen Methode aufgenommen und verarbeitet werden.

8. Gerät für rechnerunterstützte Chirurgie für eine chirurgische Operation, bei der die für Ultraschallwellen grundsätzlich nicht durchlässig Struktur einer Knochenstruktur nach die Anspruch 7 ist, wobei die Vorrichtungen zur Aufnahme der Bilddaten enthalten:
Mittel zur Aufnahme eines vorbereitenden Bilds mit grundsätzlich hoher Auflösung einer Knochenstruktur von Interesse, für eine chirurgische Operation ausgewählt,
Mittel zur Segmentierung des vorbereitenden Bildes für die Bestimmung einer Kontur der Knochenstruktur von Interesse, und
Mittel zur Aufnahme, während einer Operation, von mindestens einem digitalisierten Echographiebild einer Zone mit gleichartiger Knochenstruktur, wobei dieses Bild mit den Messungen zur Lokalisierung und Orientierung in das Bezugssystem mit einen festen Bezug eingebunden wird,
und in dem das System zur Bildverarbeitung einen Mikroprozessor mit Zugriff auf Bilddaten enthält und mit Mitteln versehen ist, um folgendes auszuführen:
eine automatischen Extraktion der Konturpunkte nach einen der Ansprüche 1 bis 5 des digitalisierten Echographiebilds für die Ausgabe der Konturpunkte von Interesse in Echtzeit während der Operation mit ihren Lokalisierungen und Orientierungen im Bezugssystem,
eine Gleichsetzung der Kontur der Knochenstruktur von Interesse des segmentierten vorbereitenden Bildes und der extrahierten Konturpunkte des Echographiebilds zur Lokalisierung und Orientierung des vorbereitenden Bilds im Bezugssystem, und in dem der Mikroprozessor auch mit Mitteln zur Übertragung an das Anzeigesystem versehen ist:
einer gleichzeitigen Darstellung des vorbereitenden Bildes, im Bezugssystem geortet, und der chirurgischen Instrumente in der Form von virtuellen Bildern in diesem Bild zur Darstellung der reellen chirurgischen Instrumente, die während der Operation verwendet werden, mit ihren aktuell im Bezugssystem bestimmten Lokalisierungen und Orientierungen.

## Claims

1. An image processing method including an acquisition step utilizing an ultrasonic transducer coupled to an echographic device, of a digitized echographic image having a structure which is substantially not transparent to the ultrasonic waves, and an extraction step for the extraction of contour points of said structure, the extraction step for the extraction of points being automated by way of the following sub-steps:
- detection (4), in columns of points of the digitized echographic image, of the points of a locally maximum intensity along each column,
- chaining (5) of the detected points from one column to another in a predetermined neighborhood,
- selection (6-10) of chains in the standard echographic image by determining the chain nearest to the ultrasonic transducer as being the contour of said structure.

2. A method as claimed in Claim 1, in which the chaining step is performed on the basis of the following criteria:
- definition of an intensity threshold for the chained points,
- definition of a chaining direction, starting from a first column towards a last column from one edge to an opposite edge of the digitized image,
- definition of the neighborhood as a reference distance in a predetermined branch in order to chain two points in different columns.

3. A method as claimed in Claim 2, in which the chaining step is performed in conformity with the following supplementary criteria:
- formation of a beginning of a new chain by means of any point that does not satisfy the preceding neighborhood criterion,
- definition of a minimum length of the chains for the saving of the chained points,
- elimination of points not belonging to any chain.

4. A method as claimed in Claim 3, in which the chaining step also includes an interpolation operation for filling gaps in the chains which gaps are smaller than a predetermined distance.

5. A method as claimed in any one of the Claims 1 to 4, in which the determination of the location of the transducer is performed in the digitized echo graphic image, showing radial ultrasonic scan lines, by determination of the intersection of two extreme radial lines in this image.

6. An image processing system including a processor (63) which has access to image data and includes means for making images acquired at different instants correspond with different image acquisition means, these means comprising instructions to effect:
a) an acquisition at a first instant with first image acquisition means of a first image which has substantially high resolution in the region having a structure of interest not substantially transparent to ultrasonic waves,
b) a segmentation of said first image to determine a contour of the structure of interest,
c) an acquisition at a second instant by means of an echographic image acquisition system by at least one digitized echographic image of a region which has the same structure of interest not substantially transparent to ultrasonic waves, this echographic image being associated with location and orientation measures in a marking system which has a fixed mark with respect to the echographic image formation system,
d) an automatic extraction of contour points as claimed in any one of the claims 1 to 5, of the digitized echographic image to produce contour points of the structure of interest with their locations and orientations in the marking system;
e) a correspondence of the contour of the structure of interest of said first segmented image and contour points extracted from the echographic image to locate and direct said first image in the marking system,
f) a simultaneous representation of said first image marked in the marking system and surgical instruments in the form of virtual images superimposed on this image representing real surgical instruments used during an operation with their actual locations and orientations determined in the marking system.

7. A device for computer-assisted surgery for a surgical operation of a structure of interest comprising:
an image data acquisition system, including a device (61) for the acquisition of high-resolution images and a device (62) for the acquisition of echographic images,
a marking system comprising a fixed marking device, a marking device for the bone structure of interest which is not substantially transparent to the ultrasonic waves, a marking device of echographic image acquisition means, and a marking device of movable instruments in the field of echographic images with respect to the fixed marking,
an image processing system as claimed in claim 6, including a processor which has access to image data and has means comprising instructions to effect a correspondence of images,
and a display system and occasionally recording system for displaying and recording image data acquired and processed by said method, linked to the image processing system.

8. A device for computer-assisted surgery as claimed in claim 7, for a surgical operation where the structure that is not substantially transparent to the ultrasonic waves is a bone structure, in which the image data acquisition devices comprise:
acquisition means for the acquisition of a pre-operation image which has a substantially high resolution of a bone structure of interest selected for a surgical operation,
segmentation means for segmenting the pre-operation image to determine a contour of the bone structure of interest,
and acquisition means for the acquisition during an operation of at least one digitized echographic image of an area of the same bone structure, this image being associated with location and orientation measures in the location system having a fixed mark,
and in which the image processing system comprises a microprocessor which has access to image data and which has means for effecting:
an automatic extraction of contour points as claimed in one of the claims 1 to 5, of the digitized echographic image to produce contour points of the bone structure of interest in real time during the operation with their locations and orientations in the marking system;
a correspondence of the contour of the bone structure of interest of the segmented pre-operation image and contour points extracted from the echographic image to locate and direct the pre-operation image in the marking system
and in which the microprocessor also comprises means for transmission to the display system;
a simultaneous representation of the pre-operation image marked in the marking system and surgical instruments in the form of virtual images superimposed on this image representing real surgical instruments used during an operation with their actual locations and orientations determined in the marking system.
